# EUROPÄISCHE PATENTSCHRIFT

(11) **EP 1 250 289 B1**
(45) Veröffentlichungstag und Bekanntmachung des Hinweises auf die Patenterteilung: **25.05.2005**
(21) Anmeldenummer: 01905684.5
(22) Anmeldetag: 24.01.2001
(51) Int. Cl.: C02F 1/00

(54) **VORRICHTUNG ZUM BEHANDELN VON WASSER**
DEVICE FOR TREATING WATER
DISPOSITIF POUR TRAITER DES EAUX

(30) Priorität: 24.01.2000 DE 10002977; 03.02.2000 DE 10004675; 11.10.2000 DE 10050489
(43) Veröffentlichungstag der Anmeldung: 23.10.2002
(73) Patentinhaber: REV22 AG, 8280 Kreuzlingen (CH)
(72) Erfinder: BARTL, Ludwig, 78464 Konstanz (CH); COOKSON, Andrew, 8280 Kreuzlingen (CH); STEFKA, Karel, 602 00 Brno (CZ)
(74) Vertreter: Behrmann, Niels, Dipl.-Ing.
(86) Internationale Anmeldenummer: PCT/EP2001/000781
(87) Internationale Veröffentlichungsnummer: WO 2001/055035

(56) Entgegenhaltungen:
- EP-A- 0 612 693
- EP-A- 0 952 117
- WO-A-00/54817
- WO-A-99/38807
- DE-A- 2 311 504
- DE-A- 2 846 452
- FR-A- 2 036 491
- US-A- 3 933 606
- US-A- 5 304 289

## Beschreibung

Die vorliegende Erfindung betrifft eine Vorrichtung zum Behandeln von Wasser nach dem Oberbegriff des Patentanspruchs 1, und zwar insbesondere eine Vorrichtung zur Aufbereitung von Wasser durch Dekontaminieren bzw. Sterilisieren.

Vor dem Hintergrund einer einfachen, handhabungsfreundlichen und portablen Vorrichtung zur Verbesserung der Trinkwasserqualität sind bislang hauptsächlich Vorrichtungen erhältlich, die auf einer Filterwirkung (z. B. durch Aktivkohlefilter) basieren, oder aber die mit chemischen Mitteln aus durch Bakterien, Schwermetallen usw. verseuchten wasserhaltigen Flüssigkeiten die Trinkwassergewinnung ermöglichen.

Allerdings haben sich im praktischen Gebrauch rein filterbasierende Lösungen für solche aufzubereitende Wassermengen, die Bakterien oder andere Mikroorganismen enthalten, als oftmals unzureichend erwiesen, und andere bekannte Verfahren gerade zum Abtöten von Mikroorganismen in Wasser, etwa die Beaufschlagung mit ultravioletter Strahlung, erweisen sich für einen Gebrauch in einem portablen Gerät, nicht zuletzt auf Grund der notwendigen Energieversorgung, als ungeeignet.

Ferner sind aus dem Stand der Technik gattungsbildende Technologien bekannt, mit Hilfe elektrischer Signale (typischerweise Gleichspannungen) Dekontaminations- bzw. Reinigungswirkungen von verschmutztem Wasser zu erreichen. Hier hängt die Wirksamkeit typischerweise davon ab, dass durch elektrolytische Wirkung sogenannte anodische Oxidanten, typischerweise Chlor, aus dem kontaminierten Wasser freigesetzt werden und es tatsächlich dann dieses Chlor ist, welches die gewünschte bakterientötende Wirkung besitzt. Üblicherweise besitzen derartige, bekannte Vorrichtungen daher auch großflächige Elektroden, um den elektrolytischen Effekt bestmöglich zur Geltung zu bringen.

Allerdings besitzt eine solche Vorgehensweise auch den Nachteil, dass das freigesetzte Chlor sich selbst wiederum negativ auf die Wasserqualität, insbesondere im Fall von Trinkwasser, auswirkt, und darüber hinaus wird Geruch und Geschmack eines so behandelten Wasser typischerweise als unangenehm empfunden.

Die DE 28 46 452 A sowie die US-A-3 933 606 offenbaren jeweils eine Vorrichtung zum antibakteriellen Behandeln, insbesondere Dekontaminieren und/oder Sterilisieren von Wasser sowie zum Abtöten von Mikroorganismen in Wasser, wobei eine solche Vorrichtung einen zum Aufnehmen einer zur Behandlung vorgesehenen Wassermenge ausgebildeten Behälter sowie eine zum Beaufschlagen der Wassermenge im Behälter ausgebildeten Elektrodenanordnung aufweist, die mit einer behälterextern vorgesehenen elektrischen Signalerzeugungsvorrichtung verbind- und betreibbar ist.

Ferner ist aus diesem Stand der Technik eine getaktete Konstantstromquelle als Signalerzeugungsvorrichtung bekannt, wobei eine Regelung der Impulsbreite vorgenommen wird. Das Wechselsignal wird in Abhängigkeit von der Leitfähigkeit des Wassers variiert.

Ferner offenbart die WO 99 38807 A eine stationäre Vorrichtung zur Wasserreinigung, wobei das zu reinigende Wasser mit Stromimpulsen beaufschlagt wird.

Aufgabe der vorliegenden Erfindung ist es daher, eine Vorrichtung zur Behandlung, insbesondere zur Aufbereitung, Dekontamination und Sterilisation von Wasser zu schaffen, die mit geringem Aufwand bedien- und betreibbar ist, portabel ausbildbar ist und darüber hinaus hinsichtlich ihrer Energieversorgung universell verwendbar ist sowie sichere, reproduzierbare und schnell erreichbare Sterilisationswirkungen ermöglicht, jedoch bei Minimierung der Erzeugung anodischer Oxidanten.

Die Aufgabe wird durch die Vorrichtung mit den Merkmalen des Patentanspruchs 1 gelöst; vorteilhafte Weiterbildungen der Erfindung sind in den Unteransprüchen beschrieben.

Erfindungsgemäß vorteilhaft wird der durch den Erfinder entdeckte Effekt ausgenutzt, dass elektrische Wechselsignale, wenn diese über eine Elektrode in eine zu behandelnde Wassermenge eingeleitet werden, einen dekontaminierenden, insbesondere Mikroorganismen abtötenden Effekt besitzen, und zwar auch dann, wenn elektrolytische Effekte weitgehend unterdrückt und damit das Entstehen von anodischen Oxidanten verhindert werden (wie dies im Rahmen der Erfindung etwa durch minimierte Oberflächen der verwendeten Elektroden erreicht werden kann).

Ein kritischer Parameter bei einer möglichst effektiven Dekontamination durch das elektrische Wechselsignal ist die erfindungsgemäß festgestellte Tatsache, dass ein optimaler Amplitudenhub des Wechselsignals sowie ein optimales Signal-/Pausenverhältnis des Signals (insbesondere bei einem Rechtecksignal) zum Erreichen einer optimalen Wirkung auf die zu beseitigenden Mikroorganismen von einem Leitwert des Wassers abhängig ist.

Es liegt daher im Rahmen der Erfindung, eine Einheit zum automatisierten Bestimmen eines Leitwertes der zu behandelnden Wassermenge vorzusehen, und abhängig von einem Ergebnis dieser Leitwertbestimmung dann eine entsprechende Signaleinstellung, insbesondere hinsichtlich der Frequenz, Signalamplitude und/oder des Signal-/Pausenverhältnisses innerhalb einer Periode des Wechselsignals vorzunehmen.

Experimentell wurde zudem festgestellt, dass eine derartige Zuordnung einem nicht-linearen Verhältnis zwischen Leitwert und maximaler Signalamplitude folgt, wobei insbesondere eine parabolische Form einer entsprechenden Einstellkurve bevorzugt ist.

Vorteilhaft erreicht die Integration des Wasserbehälters sowie der Signalerzeugungsvorrichtung für die Elektrodenanordnung in eine portable Einheit, dass damit das Gerät bedarfsabhängig, flexibel und insbesondere auch an solchen Orten verwendet werden kann, wo ein Bedarf an sauberem, dekontaminiertem Wasser besteht.

Da zudem ein portabler, flexibler Einsatz einer solchen leicht bewegbaren Einheit es erfordert, weitgehend unabhängig von einem (für große Energiemengen geeigneten) Stromversorgungsnetz zu sein, wurde zudem im Rahmen der vorliegenden Erfindung die Möglichkeit geschaffen, die elektrische Signalerzeugungsvorrichtung mit Niederspannung zu betreiben, wobei als Niederspannung im Rahmen der vorliegenden Erfindung jegliche Spannung kleiner als eine Netz-Wechselspannung verstanden werden soll, welche üblicherweise aus portablen Spannungsversorgungseinheiten, wie etwa Batterien oder dergleichen, zu gewinnen ist. Insbesondere ist unter "Niederspannung" im vorliegenden Fall daher eine Spannung von 12, 24 oder 30 V zu verstehen, oder aber eine von gängigen Solarzelleneinheiten abgegebene Spannung in dieser Größenordnung.

Im Ergebnis erreicht damit die vorliegende Erfindung, dass bei niedrigem Energieaufwand eine hochwirksame Dekontamination von verschmutztem Wasser und damit eine Umwandlung in Trinkwasser durchgeführt werden kann, wobei die erfindungsgemäß geschaffene Vorrichtung durch ihre Portabilität beliebig beweg- und an einen Einsatzort verbringbar ist.

Im Rahmen der Erfindung ist dabei unter dem "Aufnehmen" der zur Behandlung vorgesehenen Wassermenge nicht nur ein chargenweiser Betrieb zu verstehen, sondern, wie die an späterer Stelle zu beschreibenden Ausführungsbeispiele verdeutlichen, ist insbesondere auch eine Ausführung und eine Realisierung der Erfindung als Durchflussgerät beabsichtigt und von der Erfindung umfasst, wobei hier der Behälter als Durchflussbehälter ausgebildet ist.

Es hat sich herausgestellt, dass eine Beaufschlagung der Wassermenge mit einem Wechselsignal, welches eine Gleichspannungskomponente besitzt, und welches weiter bevorzugt ein gleichgerichtetes Wechselspannungssignal ist (also lediglich Signalkomponenten in einer Polarität besitzt) besonders wirksam auf Bakterien ist. Weiterbildungsgemäß ist daher die Signalerzeugungsvorrichtung zum Erzeugen eines derartigen Signalmusters mit Gleichspannungsanteil (d. h. einem hinsichtlich beider Polaritäten nicht symmetrischen Signalmuster) ausgebildet. Weiter vorteilhaft ist zudem eine Realisierungform der Erfindung denkbar, wo durch ein (periodisches) Umpolen des Elektrodensystems unerwünschte Verkalkung oder andere ungewollte Ablagerungen auf den Elektroden vermieden werden. Gemäß dieser bevorzugten Weiterbildung der Erfindung ist zudem vorgesehen, vor jedem dieser Umpolungsvorgänge eine asymmetrische, stromfreie Zeitspanne von ca. zwischen 1 und 5 sec. einzuschieben, um das Auftreten anodischer Oxidanten weiter zu verhindern. Zudem wirken sich derartige Pausen beim Beaufschlagen des kontaminierten Wassers (und unter Ausnutzen von Nachschwing- bzw. Relaxationseffekten im Wasser) positiv auf den Energieverbrauch der Anordnung, wichtig insbesondere im portablen Betrieb, aus.

Besondere Bedeutung in der praktischen Realisierung kommt zudem der Ausgestaltung der Elektrodenanordnung zu, wobei es sich weiterbildungsgemäß als besonders bevorzugt herausgestellt hat, zumindest eine Elektrode der Elektrodenanordnung mittels eines langgestreckten Leiterstücks (insbesondere Drahtes) zu realisieren, wobei dieser Draht, bei zum Zweck der Minimierung anodischer Oxidanten entsprechend minimierter Oberfläche, typische Durchmesser zwischen ca. 0,1 und 0,5 mm aufweist und geeignet aus Platin od. dgl. Materialien realisiert werden kann. Insbesondere bei dieser Elektrodengestaltung zeigt sich der prinzipielle Unterschied zu bekannten, elektrolytisch basierten Dekontaminationsverfahren, da es herkömmlicherweise ja eher auf möglichst großflächige Elektroden ankommt.

Zur ergänzenden Behandlung des Wassers im Hinblick auf Schwermetalle, Nitrate, Chlorverbindungen usw. bietet es sich an, die Vorrichtung zusätzlich mit einer Filtereinheit zu versehen, wobei, je nach Einsatzzweck und gewünschter Intensität der Filterwirkung, eine solche Filtereinheit einem Behältereinlass vor- und/oder einem Behälterauslass nachgeschaltet sein kann; auch ist es möglich, hier variable, einschwenkbare Filter usw. zu verwenden.

Im Rahmen der vorliegenden Erfindung hängt die Effektivität der Tötung von Krankheitserregern (Einzellern, Parasiten, Bakterien und Viren) im Wasser auch von einer konkreten Form des mittels der Elektrodenanordnung eingebrachten elektrischen Wechselsignals ab, wobei es sich als besonders bevorzugt herausgestellt hat, ein i. w. rechteckförmiges Wechselsignal einzubringen.

Wie wissenschaftliche Erprobungen der vorliegenden Erfindung erwiesen haben, kann eine große Anzahl von coliformen, mesophilen und psychrophilen Bakterien verschiedener Typen durch Verwendung der vorliegenden Vorrichtung in relativ kurzer Behandlungszeit, typischerweise im Bereich zwischen 5 und 15 Minuten, abgetötet werden, so daß durch die vorliegende Erfindung ein Weg geschaffen ist, auf flexible und einfache Weise, netzspannungsunabhängig und portabel Wasser zu reinigen und damit ein Infektionsrisiko, insbesondere auch in ohnehin unterversorgten Gebieten, drastisch zu verringern.

Eine besonders bevorzugte Ausführungsform der Erfindung liegt insbesondere auch darin, eine Mehrzahl der erfindungsgemäßen Behälter vorzusehen und diese modulweise so auszugestalten, dass mehrere Behälter parallel zum Aufnehmen der Wassermenge ausgebildet sind und so bei einem Durchflussbetrieb, eine Reinigungsleistung innerhalb eines vorbestimmten Zeitraumes erhöhen können, und/oder aufeinanderfolgend verschaltet werden können, um eine verlängerte Wirk- bzw. Beaufschlagungsstrecke für das Wasser zu schaffen.

Weitere bevorzugte Weiterbildungen der Erfindung sehen vor, dass die elektrische Signalerzeugungsvorrichtung im Hinblick auf Betriebszeiten des Erzeugens des elektrischen Wechselsignals programmierbar ist, wobei insbesondere auch voreingestellte Programme mit Signal- und/oder Betriebszeitmustern abspeicher- und geeignet aufrufbar sind.

Eine weitere bevorzugte Weiterbildung der Erfindung besteht darin, zumindest einige Elektroden der Elektrodenanordnung, weiter bevorzugt solche, die benachbart eines Behälterauslasses vorgesehen sind, als (weiter bevorzugt austauschbare) Magnesiumelektroden zu realisieren, um so eine zusätzliche, gesteuerte Beaufschlagung des erfindungsgemäß behandelten Wassers mit Magnesiumionen (geeignet wären etwa 5 - 15 mg Mg pro Liter Wasser) zu ermöglichen.

Für diesen Zweck eignet es sich generell, Magnesiumelektroden zu verwenden, insbesondere im Hinblick auf eine in der Wassermenge bereits vorhandene Konzentration von Chloridionen: Durch (aus den Magnesiumelektroden austretende) Magnesiumionen findet insoweit eine Neutralisierung des Chlorides statt, so dass, insbesondere angesichts permanent steigender Chloridbelastungen von Trinkwasser (und entsprechend steigender Grenzwerte) eine effektive Behandlung des Chloridproblems im Rahmen der vorliegenden Erfindung möglich ist.

In diesem Zusammenhang ist auch eine weitere, vorteilhafte Weiterbildung der Erfindung zu betrachten, welche, als Reaktion auf einen vorab durch ansonsten bekannte Mittel (etwa durch Teststreifen) diagnostizierten, erhöhten Chloridgehalte, einen manuellen Eingriff in die (im Rahmen der Erfindung hier ansonsten automatisch ablaufende Signalerzeugung durch die Einstellmittel) ermöglicht: Durch einen solchen Eingriff könnte insbesondere eine Absenkung der Maximalamplitude und/oder eine Verkürzung der Signaldauer (im Vergleich zur Pausendauer) des elektrischen Wechselsignals erreicht werden, mit dem Zweck, die durch das Wechselsignal bewirkte Freisetzung von Chlorid zu verringern.

Eine alternative Lösungsmöglichkeit für das Chlorproblem im Rahmen der vorliegenden Erfindung liegt darin, weiterbildungsgemäß eine zusätzliche Elektrodenanordnung vorzusehen, die mit Elektroden typischerweise aus Kohle, Magnesium od.dgl. (insbesondere aus in Trinkwasser erlaubten Metallen) in Form von Stäben, einem Netz, Beschichtungen oder Platten, und durch ein separates Spannungssignal, bevorzugt Gleichspannung im Bereich zwischen 1 und 50 V mit Idealwert 25 V, beaufschlagt wird, und so für eine vorteilhafte Neutralisierung chloridhaltigen Wassers sorgt. Durch eine solche Maßnahme könnte insbesondere auch ein vorstehend beschriebener, weiterbildungsgemäß manuell möglicher Eingriff in die Arbeitsweise der erfindungsgemäßen Einstellmittel unnötig werden. Geeignet sind solche Elektroden der zusätzlichen Elektrodenanordnung ca. 1 bis 20 mm, ideal 10 mm, voneinander beabstandet, liegen als Platten von Abmessungen von ca. 10 x 100 mm vor und werden mit einem Strom (Gleichstrom oder Wechselstrom) im Bereich zwischen 20 und 100 mA, bevorzugt 20 mA, angesteuert.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1:: eine schematische Seitenansicht der erfindungsgemäßen Vorrichtung zur Wasserbehandlung;
- Fig. 2:: verschiedene Signalformdiagramme (als Funktionen der Signalspannung über der Zeit) des über die Elektrodenanordnung in die zu beaufschlagende Wassermenge einzuleitenden Wechselsignal und
- Fig. 3:: eine weitere Ausführungsform der vorliegenden Erfindung.

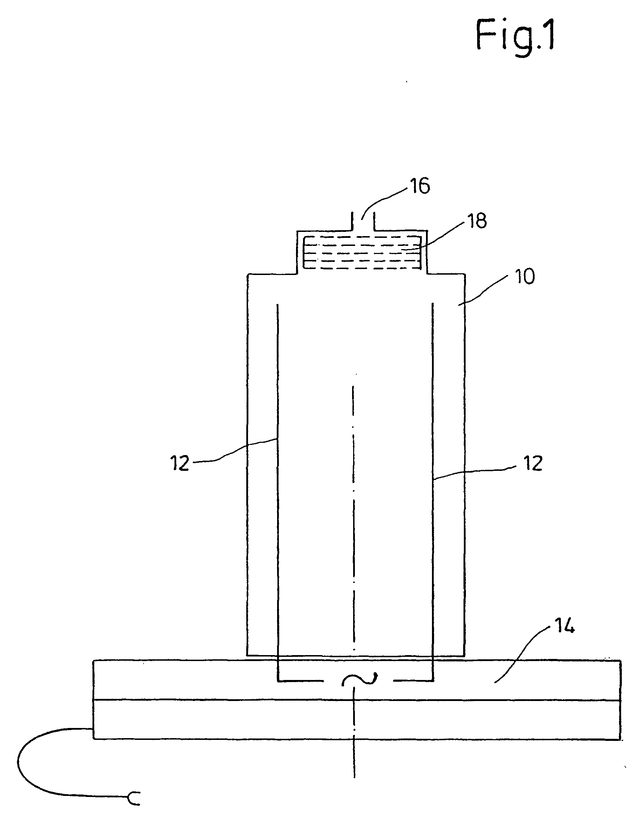

Wie in der Fig. 1 schematisch gezeigt, besteht die erfindungsgemäße Vorrichtung zum Behandeln, insbesondere Dekontaminieren von Wasser mit dem Zweck der Trinkwassererzeugung aus einer zylindrischen Behältereinheit 10, in deren Innerem eine Elektrodenanordnung bestehend aus einem Paar von Draht- bzw. Stabelektroden 12 aus Platin (Durchmesser 0,1 mm) vorgesehen und so kontaktierbar ist, dass die Elektroden 12 bodenseitig mit einem -- schematisch gezeigten -- Wechselspannungssignal beaufschlagbar sind.

Genauer gesagt wird dieses Wechselspannungssignal durch eine schematisch gezeigte Signalerzeugungsvorrichtung 14 generiert, die selbst mit einer Niedervolt-Versorgungsspannung, typischerweise einem 12V-Autobatterieanschluss od. dgl., verbindbar ist.

An ihrem der Signalerzeugungseinheit 14 entgegengesetzten oberen Einlassende 16 ist zudem schematisch eine Filtereinheit 18 gezeigt, die durch den Einlass 16 eintretendes, kontaminiertes Wasser in ansonsten bekannter Weise von Schwermetallionen, Chlor- oder Stickstoffverbindungen befreit und das gesamte Reinigungsergebnis verbessert (alternativ ist es problemlos möglich, den Behälter durch Vorsehen eines entsprechenden, nicht gezeigten Auslasses zu einem Durchflussbehälter auszubilden, so dass die Behandlung nicht chargenweise erfolgt, sondern im Wege eines permanenten Zu- und Abflusses durch den Ein- bzw. Auslass.)

In der Fig. 1 gezeigten Weise bildet die Dekontaminationsvorrichtung so eine probate, leicht auch manuell handzuhabende Einrichtung, die damit beliebig an entsprechende Einsatzorte verbracht werden kann. Typische Behältervolumina der Behältereinheit 10 liegen im Bereich zwischen etwa 0,5 Liter und etwa 5 Liter.

Die Funktionsweise der in Fig. 1 gezeigten Vorrichtung ist wie folgt: Der Benutzer füllt zu dekontaminierendes Wasser, welches neben Schwermetallionen wie Blei oder Kupfer auch Mikroorganismen in Form von Bakterien, Viren oder anderen potentiell schädlichen Erregern enthält, über den Einlass ein. Das Wasser tritt durch die Filtereinheit 18 hindurch und wird dort in ansonsten bekannter Weise gefiltert und sammelt sich in dem Inneren der Behältereinheit 10, wobei besonders bevorzugt die weitere Wasserbehandlung erst durchgeführt wird, wenn die Behältereinheit 10 vollständig gefüllt ist und damit die Stabelektroden 12 unterhalb des Wasserspiegels im Behälter 10 liegen.

Durch Aktivieren der Signalerzeugungseinheit 14 wird daraufhin ein elektrisches Wechselsignal erzeugt und an das Elektrodenpaar 12, 12 angelegt, mit der Wirkung, dass in dem die Elektroden 12 umspülenden Fluid sich ein elektrisches Feld aufbaut, welches, unter Berücksichtigung des dielektrischen Beitrags des Wassers, eine der Elektrodengeometrie sowie der Signalform des eingetragenen Wechselsignals folgende Feldausbreitung erzeugt.

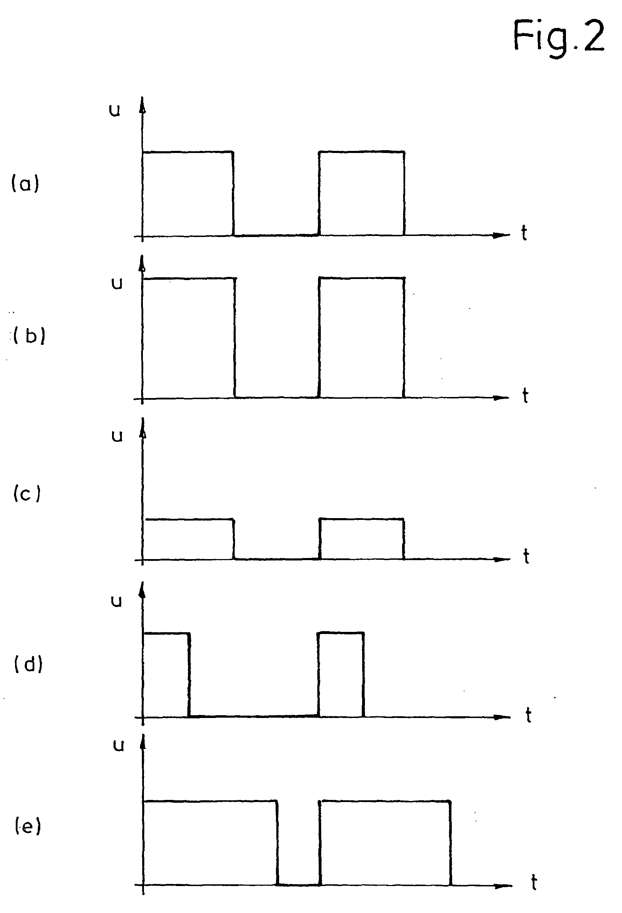

Fig. 2 zeigt verschiedene Möglichkeiten der Eintragung des Wechselsignals in das im Behälter 10 befindliche Fluid. Die mit (a) bis (e) in Fig. 2 bezeichneten Signalformen stellen dabei sämtlichst gleichgerichtete, rechteckförmige Wechselsignal dar, setzen also im dargestellten Ausführungsbeispiel lediglich eine unipolare Signalform (allerdings ist die vorliegende Erfindung weder auf die dargestellte rechteckförmige Signalform, noch auf die Unipolarität beschränkt).

Wie zudem die verschiedenen Signalmuster (a) bis (e) der Fig. 2 verdeutlichen, liegt es im Rahmen der Ausführungsform der Figur 1, die Signalform bevorzugt automatisiert einstellbar zu machen, und zwar abhängig von einem konkreten Leitwert des im Behälter befindlichen Fluids; geeignet wird dieser Leitwert vor einer Beaufschlagung des Fluids mit dem Wechselsignal oder kontinuierlich während einer solchen Beaufschlagung durch einen Messvorgang mit einer (in den Figuren nicht gezeigten) Einheit ermittelt.

In Abhängigkeit von dem so ermittelten Leitwert findet dann eine Bestimmung einer für ein solches Fluid optimalen Signalform (Signalmuster) statt, wobei im Rahmen einer bevorzugten Ausführungsform der Erfindung von einem nicht-linearen, gleichwohl stetigen (insbesondere parabolischem)Zusammenhang zwischen Leitwert und Maximalamplitude des Wechselsignals ausgegangen wird.

Konkret kann, wie etwa anhand der Beispiele (a)-(c) der Figur 2 gezeigt, eine automatische, leitwertabhängige Variation des eingetragenen Wechselsignals dadurch erfolgen, dass lediglich die Maximalamplitude des Wechselsignals verändert wird, während das Signal-/Pausenverhältnis des Signals unverändert bleibt; typischerweise könnte die so einstellbare Maximalamplitude zwischen etwa 3 V (Minimum) und etwa 50 V (Maximum) liegen, wobei praktischerweise, nicht zuletzt auch begrenzt durch die Möglichkeiten des aus Niederspannung erzeugten Eingangssignals, Maximalamplituden von 12 oder 24 V gewählt werden. Eine beispielhafte maximale Spannungsamplitude bei kleiner Leitfähigkeit (etwa im Bereich zwischen 180 µ-Scm⁻¹ und 360 µScm⁻¹) beträgt etwa 30 Volt, bei Spannungsimpulsen einer Breite von 15 µs. Eine höhere Wasserleitfähigkeit (typischerweise im Bereich zwischen 1.500 µScm⁻¹ und 2000 µScm⁻¹) würde gemäß diesem Beispiel die Spannungsamplitude automatisch auf einen Wert von etwa 10 Volt senken, mit der Folge einer signifikanten und automatischen Senkung des Durchschnittsstromes bei höherer Wasserleitfähigkeit gemäß der Erfindung.

Ergänzend oder alternativ ist es möglich, vgl. Fig. 2 (d) oder (e), anstelle der maximalen Signalamplitude (oder ergänzend dazu) das Signal-/Pausenverhältnis zu verändern, etwa dadurch, dass, wie in Fig. 2(e) gezeigt, nicht mehr die Signalzeit innerhalb einer Periode der Pausenzeit entspricht (und damit in dem Signal nicht mehr die in (a) bis (c) ablesbare Grundschwingung, bestimmt durch einen Signalimpuls, enthalten ist). Beispielhafte Werte für Pausenzeiten zwischen Impulsen betragen etwa 5 µs (bei niedriger Wasserleitfähigkeit) und bis zu 200 µs(bei hoher Wasserleitfähigkeit), bei typischen Impulsbreiten der Spannungsimpulse von ca. 15 µs.

Nach Messung der Leitfähigkeit wird gemäß eines bevorzugten Ausführungsbeispiel bei relativ hochleitfähigem Wasser (z. B. hohe Konzentrationen von Ca- oder Mg-Ionen) die Signalamplitude bis auf eine festgestellte, untere Grenzampliture (Grenzspannungswert), ggf. zuzüglich eines Sicherheitszuschlages, gesenkt. Eine derartige Minimalspannung wurde -- behälter- bzw. einsatzspezifisch -- durch Experimente ermittelt und mikrobiologisch getestet, wobei weitere Einflussgrößen einer derartigen Grenzspannung (Grenzamplitude) die konkrete Behälterform, die Wassermenge sowie Elektrodenparameter sind, etwa Form, Material und Fläche der Elektroden. Nach Einstellung der Maximalamplitude erfolgt die Einstellung des Signal-/Pausenverhältnis des Wechselsignals, wobei, wie am Beispiel oben beschrieben, bei relativ hochleitfähigem Wasser eine Verengung der Signaldauer und/oder eine Verbreitung der Pausendauer im Signal-Zeitdiagramm erfolgt.

Dagegen führt die Messung einer niedrigen Leitfähigkeit des Wassers zu einer Spannungserhöhung (d. h. Erhöhung der Maximalamplitude des Wechselsignals) sowie einer Verbreiterung der Signaldauer relativ zur Pausendauer im Signal-Zeitdiagramm. In Grenzfällen ist es möglich, dass das zu behandelnde Wasser eine derart geringe Leitfähigkeit aufweist, dass eine weitere Elektrode hinzugeschaltet werden muss, oder aber dem Wasser sind Ionen zur Leitfähigkeitserhöhung, etwa durch Salzen, hinzuzufügen. Gemäß einer besonders bevorzugten Ausführungsform werden auch diese Leitfähigkeitszustände bzw. Grenzzustände durch eine geeignete Signalisierung, etwa ein Lichtsignal, angezeigt.

Während, wie dargelegt, bevorzugt die Einstellung des Signalmusters gemäß Fig. 2 durch automatische Regelung und Einstellung einer geeigneten Mess- und Einstellelektronik erfolgen kann, ist es alternativ natürlich auch möglich, manuell aus einigen voreingestellten Signalformen zu wählen, mit Hilfe digitaler Technik aus einer Tabelle voreingestellte Signalformen zu wählen, oder andere Wege zur Anpassung der Wechselsignalform an einen konkreten Leitwert vorzunehmen. Ergänzend oder alternativ ist es möglich, innerhalb eines Behandlungsvorganges auch die Signalfrequenz zu variieren, etwa kontinuierlich zwischen einer unteren und einer oberen Grenzfrequenz. Hierdurch kann einer Frequenzabhängigkeit der Dekontaminationswirkung auf verschiedene Bakterien Rechnung getragen werden.

Eine typische Behandlungsdauer der in Fig. 1 aufgenommenen Wassermenge eines Volumens von 2 Litern liegt im Bereich zwischen etwa 2 Minuten und etwa 20 Minuten; je nach Kontaminierungsgrad sollten jedoch auch Sicherheitszuschläge hinzugerechnet werden. Eine besonders bevorzugte Weiterbildung der Ausführungsform gem. Fig. 1 liegt zudem darin, eine (nicht gezeigte) Timer- bzw. Zeitgebereinheit vorzusehen, die, idealerweise mit optischer oder anderer Signalausgabe, einem Benutzer signalisiert, sobald die vorgewählte Dekontaminationszeit der Signaleinleitung abgelaufen ist.

In der praktischen Erprobung der vorliegenden Erfindung hat sich dabei das erfindungsgemäße Prinzip nicht nur auch Bakterien vom Typ E-Coli, Salmonella, Legionella, Enterocos, Pseudamonasas Aerogenosa, Staphylococcus aureus usw. als wirksam erwiesen, auch wird davon ausgegangen, dass weitere Einzeller, Parasiten, Bakterien und Viren auf die beschriebene Art und Weise im Wasser getötet bzw. unschädlich gemacht werden.

Ebenfalls wird durch die beschriebene Vorrichtung mit Filterunterstützung erreicht, dass Schwermetallionen, Blei, Kadmium, Zink, Kupfer, Arsen usw. sowie Nitrate, Sulfate, Kohlenwasserstoffe, Chlor, organische Chlorverbindungen, Pestizide usw. entfernt werden können.

Gemäß einer weiteren, bevorzugten Ausführungsform der Erfindung ist vorgesehen, die erfindungsgemäße Behältereinheit (etwa Einheit 10 in Figur 1) selbst mit einem (nicht leitenden) Filtermaterial vollständig oder teilweise zu füllen, so dass die Behältereinheit, neben ihrer durch die Elektroden bzw. die elektrischen Signale bewirkten Dekontaminationswirkung, zusätzlich als Filter wirkt.

Gemäß einer exemplarischen Realisierungsform dieser Ausbildung der Erfindung wird geschüttetes, ggf. geeignet gesintertes Filtermaterial einer Körnung von etwa 0,5 bis 1,5 mm in den Behälter gemäß Figur 1 gefüllt, und die Behandlung des Wassers wurde in der vorbeschriebenen Weise durchgeführt. Das in den Behälter gefüllte kontaminierte Wasser wurde nach Behandlungsende als vollständig dekontaminiert entnommen, zudem konnten in dem verwendeten Filtermaterial keine lebenden Bakterien festgestellt werden. Diese Weiterbildung der Erfindung scheint daher beachtliches Potential insbesondere auch für die Dekontamination von Filtereinheiten zu besitzen, die ja bekanntermaßen Brutstätten für Bakterien sind, sofern nicht konkrete Maßnahmen hiergegen, etwa Oberflächenversilbern der Körner, vorgenommen werden. Allerdings gilt es bei der vorliegenden Erfindung darauf zu achten, dass das in die Behältereinheit eingefüllte Filtermaterial nicht zu einer Beeinflussung der elektrischen Wirkung führt, etwa durch Eigenleitfähigkeit des Filtermaterials (hier bietet z. B. Aktivkohle potentielle Probleme).

In besonders vorteilhafter Weise bietet es sich daher an, (Aktivkohle-)Filtereinheiten oder dergl. der vorliegenden Erfindung nachzuschalten, denn nicht nur sollten die hervorragenden Behandlungs- bzw. Dekontaminationseigenschaften der vorliegenden Erfindung dafür sorgen, dass eine Bakterienansammlung und damit Kontamination in der nachgeschalteten Filtereinheit vermieden werden kann, auch ist in praktischen Erprobungen der vorliegenden Erfindung ein gewisses, über den erfindungsgemäßen Behälter hinaus wirkendes Nachwirken des Behandlungseffektes zu beobachten gewesen, mit der Folge, dass offenbar auch eine unmittelbare Behandlungswirkung in einer nachgeschalteten Filtereinheit, mit den positiven Wirkungen auf Bakterien darin, erreicht wurde.

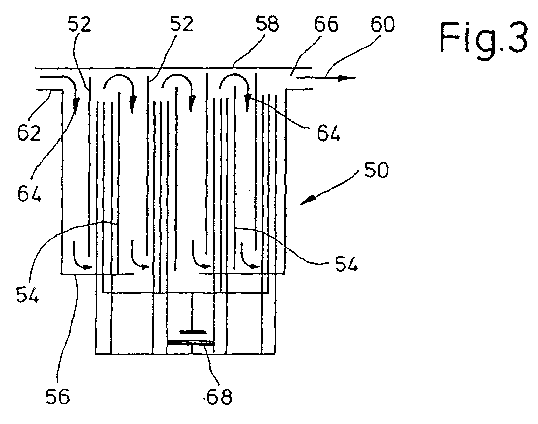

Unter Bezug auf die Fig. 3 soll nunmehr eine weitere, bevorzugte Ausführungsform der vorliegenden Erfindung beschrieben werden, die sich insbesondere auch für eine flexible Modulbauweise und -verwendung der vorliegenden Erfindung und damit zur Anpassung an verschiedene Betriebsverhältnisse eignet:

So zeigt die Fig. 3 einen quaderförmigen Behälterkörper 50, der, wie in der Seitenansicht der Fig. 3 erkennbar ist, aus einer Mehrzahl von Kammern gebildet ist, welche durch sich in der Figur vertikal erstreckende, abwechselnd von einem Behälterboden 56 bzw. einer Behälterdecke 58 in den Innenraum des Behälters 50 hineinragende Zwischenwände 52, 54 voneinander abgegrenzt sind. Genauer gesagt wechseln in der in Fig. 3 mit dem Pfeil 60 bezeichneten Strömungsrichtung abwärts gerichtete bzw. aufwärts gerichtete Zwischenwände 52, 54 einander ab, so dass im linksseitigen Einlassbereich 62 in den Behälter 50 eintretendes, kontaminiertes Wasser entlang einer durch die Pfeile 64 bezeichneten mäanderförmigen Richtung durch einander benachbarte Kammern, abwechselnd auf- und abwärts gerichtet, bis zu einem Behälterauslass 66 bewegt wird. Zusätzlich ist in der Fig. 3 zu erkennen, dass, zum Zweck des Austritts üblicher Gas- und Luftblasen, die abwärts gerichteten Zwischenwände 52 nicht durchgängig an der Deckfläche 66 des Behälters 50 ansitzen, sondern einen geringen Zwischenraum für den Gasdurchlass ermöglichen. Die Entlüftung dient vor allem auch dazu, das (in kleinsten Mengen nicht verhinderbare) Vorliegen von anodischen Oxidanten problemlos zu entlüften.

Wie die Fig. 3 zusätzlich zeigt, sind einige der auf die vorstehend beschriebene Weise gebildeten Kammern mit einem Elektrodensystem versehen, und zwar dergestalt, dass jeweils drei als Platindrähte eines Durchmessers von bevorzugt 0,1 bis 0,2 mm ausgebildete Elektroden in diejenigen Zwischenräume des Behälters 50 hineinragen, welche gemäß Pfeilrichtung 64 von aufwärts strömendem Wasser durchströmt werden. Wie in der Fig. 3 gezeigt, sind die Elektrodendrähte mit einer symbolisch gezeigten Spannungsquelle 68 geschaltet und erzeugen Signale in der oben beschriebenen Form. In der in Fig. 3 gezeigten Schaltungskonfiguration sind dabei die außenliegenden Elektrodendrähte mit einem ersten Pol verbunden, während der innenliegende Elektrodendraht mit dem anderen Pol der Signalquelle 68 verbunden ist.

Insbesondere im Hinblick auf positive Betriebs- und Reinigungseigenschaften hat sich dabei die in Fig. 3 gezeigte Konfiguration besonders bewährt, indem nämlich - überraschend - die Elektroden in den aufwärts gerichteten Wasserstrom platziert wurden.

Während im gezeigten Ausführungsbeispiel (reine) Platindrähte als Elektroden verwendet wurden, bieten sich auch andere Elektrodenformen an, so etwa Graphitstäbe oder - minen, die, bevorzugt austauschbar kontaktiert, typische Durchmesser im Bereich zwischen 0,1 und 2 mm, bevorzugt ca. 0,5 mm, aufweisen können.

Gemäß einer besonders bevorzugten, alternativen Ausführungsform ist es zudem möglich, Elektroden unmittelbar auf Wände des gezeigten Behälters (oder einer anderen Behälteranordnung) aufzubringen, und zwar durch geeignete Metallisierung (oder eine andere Art der Leiterbefestigung) auf den Behälterinnenwänden so, dass diese nicht nur mechanisch den Wasserbehälter begrenzen, 'sondern zugleich als Träger für die Elektroden dienen. Insbesondere im Hinblick auf eine günstige, automatisierbare Fertigung der Behälteranordnungen, weiter bevorzugt in einer Modulbauweise, bieten sich derartige Realisierungsformen an, wobei etwa in ansonsten bekannter Weise geeignet in der Elektroden- bzw- Leiterbahnstruktur bemusterte Glasplatten als Wände des Behältergehäuses konfiguriert werden.

In einer Weiterbildung des Modulgedankens bietet es sich zudem an, etwa analog der in Fig. 3 gezeigten Ausführungsform gebildete Behälter (die typischerweise von 3 bis 30 cm lang und 5 bis 15 hoch sein und eine typische Dicke von 20 bis 50 mm aufweisen können) so auszubilden, dass eine Mehrzahl dieser Behälter als Module entweder parallel (und damit gleichzeitig) mit einfliessendem Wasser beschickt oder aber benachbarte Behältermodule aufeinanderfolgend von dem zu reinigenden Wasser durchströmt werden können. Durch diese Technologie ist es dann insbesondere möglich, eine größere Anzahl von Behältern in einer standardisierten Größe einfach und kostengünstig herzustellen und dann durch geeignete Anordnung einer zu bestimmenden Mehrzahl von derartigen Modulen eine für eine geeignete Reinigungssituation und -leistung notwendige Anzahl von Modulen zusammenzustellen, ohne dass jeweils individuelle Behältergrößen hergestellt werden müssen.

Gemäß weiterer, bevorzugter Ausführungsformen der Erfindung sind zahlreiche Modifikationen möglich: So ist die Behälterform nahezu unbeschränkt, sie kann, neben der gezeigten zylindrischen Form, auch quaderförmig, ellipsenförmig usw. sein. Die Elektroden können neben den gezeigten Positionen im Behälterinneren, auch unmittelbar Teile der Behälterwand sein, so etwa als Netz (z. B. aus Kohlefasern realisiert) direkt auf die Behälterinnenwand aufgebracht sein, alternativ etwa als Platinfolie.

Eine weitere, alternative Realisierungsform der Erfindung erfolgt durch Trennen der Elektroden vom Medium, etwa durch entsprechendes Membranmaterial (ionendurchlässiges Material).

Experimentell wurde zudem festgestellt, dass sich die erfindungsgemäße Dekontaminationswirkung durch Druckeinwirkung oder durch Blasen für einen vorbestimmten Zeitraum erhöhen lässt.

Starke Leitfähigkeitsunterschiede des Wassers lassen sich, neben Einbringen von zusätzlichen Ionen durch Kochsalz, Kalzium oder Magnesiumsalzen besser behandelbar machen; hohe Wasserleitfähigkeit verlangt möglicherweise spezielle Elektrodenformen.

Sollte das zu reinigende Wasser in Extremfällen stark biologisch verunreinigt sein, ist es im Rahmen der Erfindung möglich, weitere Klärungs-, Flockungs- und/oder Durchlüftungsstufen sowie Filtrationsschritte vor- oder nachzuschalten.

Hervorzuheben bleibt letztendlich, dass durch die vorliegende Erfindung, wie beabsichtigt, das Auftreten von anodischen Oxidanten, insbesondere Chlor, weitestgehend verhindert werden konnte. Nicht zuletzt ist dieser Effekt auch das Ergebnis der erfindungsgemäße, automatischen Einstellung und Veränderung der Wechselsignale in Abhängigkeit von einer aktuellen Wasserleitfähigkeit, was vorteilhaft insbesondere auch dazu führt, dass bei Realisierung der Erfindung als Durchflusssystem das Wasser sofort nach einem Behandlungsdurchlauf ohne ein Sorbieren von freiem Chlor in Aktivkohlefiltern od. dgl. trinkbar ist. Experimentelle Überprüfungen des Chlorgehaltes bzw. des Entstehens von Chlor durch die erfindungsgemäße Behandlung haben dazu geführt, dass bei bakteriell kontaminiertem Wasser mit typischerweise geringen Mengen von 1 mg Chloriden pro Liter Wasser überhaupt kein freies Chlor gemessen wurde; bei typischen (normalen) Gehalten von ca. 10 mg Chloriden pro Liter Wasser wurde nach der Behandlung ebenfalls kein freies Chlor festgestellt, und lediglich bei bakteriell kontaminiertem Wasser mit starkem Chloridgehalt (mit 120 mg Chloriden pro Liter Wasser sogar noch oberhalb der Normgrenze von 100 mg/Liter) wurde nach erfindungsgemäßer Behandlung ein freier Chlorgehalt im Auslauf < 0,1 mg pro Liter festgestellt, was ein Vielfaches unterhalb einem erlaubten Chlorgehalt für Trinkwasser liegt.

## Patentansprüche

1. Vorrichtung zum antibakteriellen Behandeln, insbesondere Dekontaminieren und/oder Sterilisieren von Wasser sowie zum Abtöten von Mikroorganismen in Wasser, mit
einem zum Aufnehmen einer zur Behandlung vorgesehenen Wassermenge ausgebildeten Behälter (10) und einer zum Beaufschlagen der Wassermenge im Behälter ausgebildeten Elektrodenanordnung (12), die mit einer behälterextern vorgesehenen elektrischen Signalerzeugungsvorrichtung (14) verbind- und betreibbar ist, wobei die elektrische Signalerzeugungsvorrichtung (14) mit Niederspannung betreibbar und zum Erzeugen eines elektrischen Wechselsignals zwischen Elektroden der Elektrodenanordnung mit einer Maximalamplitude < 50 V und einer Signalfrequenz im Bereich zwischen 1 und 5000 kHz, insbesondere 5 bis 50 kHz, ausgebildet ist, die Signalerzeugungsvorrichtung Einstellmittel aufweist, die zum automatischen Verändern einer Maximalamplitude, eines Amplitudenhubs und/oder eines Signal-/Pausenverhältnisses des Wechselsignals, abhängig von einem Leitwert der Wassermenge, ausgebildet sind, der Behälter und die Signalerzeugungsvorrichtung eine portable Einheit bilden und die Elektrodenanordnung mindestens ein zumindest abschnittsweise langgestrecktes, drahtförmiges oder stabförmiges oder als Bemusterung auf einer Innenwand des Behälters konfiguriertes, Leiterstück als Elektrode (12) aufweist.

2. Vorrichtung nach Anspruch 1, **dadurch gekennzeichnet, dass** das elektrische Wechselsignal eine Gleichspannungskomponente besitzt und bevorzugt ein gleichgerichtetes Wechselspannungssignal ist.

3. Vorrichtung nach Anspruch 1 oder 2, **dadurch gekennzeichnet, dass** der Behälter und die Signalerzeugungsvorrichtung eine manuell handhabbare Einheit bilden.

4. Vorrichtung nach einem der Ansprüche 1 bis 3, **dadurch gekennzeichnet, dass** die Elektrodenanordnung drahtförmige Elektroden eines Maximaldurchmessers von 0,5 mm, bevorzugt 0,1 mm, aufweist.

5. Vorrichtung nach einem der Ansprüche 1 bis 4, **dadurch gekennzeichnet, dass** die Elektrodenanordnung aus einem Magnesiummaterial so realisiert ist, dass, insbesondere bei einem vorhandenen Chloridgehalt in der zur Behandlung vorgesehenen Wassermenge, bei der Behandlung Magnesiumionen in die Wassermenge treten können.

6. Vorrichtung nach einem der Ansprüche 1 bis 5, **dadurch gekennzeichnet, dass** die Einstellmittel so ausgebildet sind, dass bei einem niedrigen Leitwert der Wassermenge eine Spannungsamplitude des Wechselsignals erhöht und bei einem höheren Leitwert die Spannungsamplitude vermindert wird.

7. Vorrichtung nach einem der Ansprüche 1 bis 6, **dadurch gekennzeichnet, dass** die Einstellmittel so ausgebildet sind, dass bei einem niedrigeren Leitwert der Wassermenge ein zeitlicher Abstand zwischen aufeinanderfolgenden Impulsen des Wechselsignals auf einen niedrigen Wert eingestellt, und bei einem höheren Leitwert der zeitliche Abstand auf einen höheren Wert eingestellt wird.

8. Vorrichtung nach einem der Ansprüche 1 bis 7, **gekennzeichnet durch** eine einem Behältereinlass des Behälter vorgeschaltete und/oder einem Behälterauslass des Behälters nachgeschaltete Filtereinheit.

9. Vorrichtung nach einem der Ansprüche 1 bis 8, **dadurch gekennzeichnet, dass** der Behälter zumindest teilweise mit einem Filtermaterial gefüllt ist und selbst als Filter wirkt, wobei die im Behälter vorgesehenen Elektrodenanordnung zur Dekontamination bzw. Sterilisation des als Filter wirkenden Behälters wirkt.

10. Vorrichtung nach einem der Ansprüche 1 bis 9, **dadurch gekennzeichnet, dass** der Behälter als Durchflussbehälter ausgebildet ist.

11. Vorrichtung nach einem der Ansprüche 1 bis 10, **gekennzeichnet durch** Mittel zur elektronischen Bestimmung eines elektrischen Leitwerts der Wassermenge, wobei die Mittel so mit der Signalerzeugungsvorrichtung verbunden sind, dass als Reaktion auf einen vorbestimmten Leitwert die Signalerzeugungsvorrichtung ein zugehöriges Signalmuster des elektrischen Wechselsignals einstellt und ausgibt.

12. Vorrichtung nach einem der Ansprüche 1 bis 11, **gekennzeichnet durch** eine zusätzliche, mit einer separat anlegbaren Gleich- oder Wechselspannung beaufschlagbaren Zusatzelektrodenanordnung, welche Kohlenstoff, Magnesium und/oder ein Edelmetall aufweist und so angeordnet ist, dass **durch** deren Wirkung anodische Oxidanten, insbesondere freies Chlor und Chlor-Verbindungen, in der Wassermenge neutralisier- bzw. eliminierbar sind.

13. Vorrichtung nach einem der Ansprüche 1 bis 12, **dadurch gekennzeichnet, dass** eine Mehrzahl von bevorzugt quaderförmigen Behältern modulartig vorgesehen und so konfigurierbar ist, dass die zur Behandlung vorgesehene Wassermenge eine Mehrzahl der modulartigen Behälter gleichzeitig durchströmen, oder die Mehrzahl der modulartigen Behälter sequenziell durchströmen kann.

14. Vorrichtung nach einem der Ansprüche 1 bis 13, **dadurch gekennzeichnet, dass** die elektrische Signalerzeugungsvorrichtung mit einer bevorzugt programmierbaren Zeitsteuereinheit verbunden ist, die zum vorbestimmten Einstellen von Betriebszeiten der elektrischen Signalerzeugungsvorrichtung ausgebildet ist.

## Claims

1. Device for the antibacterial treatment, in particular decontamination and/or sterilisation of water and for the killing of microorganisms in water, having a container (10) designed for receiving a quantity of water provided for treatment and an electrode arrangement (12) designed to act on the quantity of water in the container and which can be connected and operated with an electrical signal-generating device (14) provided externally of the container, wherein the electrical signal-generating device (14) can be operated at low voltage and is designed for the generation of an electrical alternating signal between electrodes of the electrode arrangement having a maximum amplitude < 50 V and a signal frequency in the range between 1 and 5,000 kHz, in particular 5 to 50 kHz, the signal-generating device has adjusting means which are designed for the automatic changing of a maximum amplitude, an amplitude stroke and/or a signal/pause ratio of the alternating signal, depending on a conductance of the quantity of water, the container and the signal-generating device form a portable unit and the electrode arrangement has at least one conductor element as electrode (12) which is elongated at least in sections, wire-like or rod-like or configured as a sample on an inner wall of the container.

2. Device according to claim 1, **characterised in that** the electrical alternating signal has a direct voltage component and is preferably a rectified alternating voltage signal.

3. Device according to claim 1 or 2, **characterised in that** the container and the signal-generating device form a unit which can be handled manually.

4. Device according to one of claims 1 to 3, **characterised in that** the electrode arrangement has wire-like electrodes of a maximum diameter of 0.5 mm, preferably 0.1 mm.

5. Device according to one of claims 1 to 4, **characterised in that** the electrode arrangement is realised from a magnesium material so that, in particular for an existing chloride content in the quantity of water provided for treatment, magnesium ions may enter the quantity of water during the treatment.

6. Device according to one of claims 1 to 5, **characterised in that** the adjusting means are designed so that for a low conductance of the quantity of water, a voltage amplitude of the alternating signal is increased and for a higher conductance, the voltage amplitude is reduced.

7. Device according to one of claims 1 to 6, **characterised in that** the adjusting means are designed so that for a lower conductance of the quantity of water, a time interval between sequential pulses of the alternating signal is adjusted to a low value, and for a higher conductance, the time interval is adjusted to a higher value.

8. Device according to one of claims 1 to 7, **characterised by** a filter unit connected upstream of a container inlet of the container and/or downstream of a container outlet of the container.

9. Device according to one of claims 1 to 8, **characterised in that** the container is filled at least partly with a filter material and itself acts as a filter, wherein the electrode arrangement provided in the container acts for the decontamination or sterilisation of the container acting as a filter.

10. Device according to one of claims 1 to 9, **characterised in that** the container is designed as a throughflow container.

11. Device according to one of claims 1 to 10, **characterised by** means for the electronic determination of an electrical conductance of the quantity of water, wherein the means are connected to the signal-generating device so that the signal-generating device adjusts and emits an associated signal pattern of the electrical alternating signal as a reaction to a predetermined conductance.

12. Device according to one of claims 1 to 11, **characterised by** a further additional electrode arrangement which can be acted on by a direct or alternating voltage which can be applied separately and which has carbon, magnesium and/or a noble metal and is arranged so that as a result of its action, anodic oxidants, in particular free chlorine or chlorine compounds, can be neutralised or eliminated in the quantity of water.

13. Device according to one of claims I to 12, **characterised in that** a plurality of preferably cuboid containers are provided like modules and can be configured so that the quantity of water provided for treatment flows through a plurality of module-like containers at the same time, or may flow through the plurality of module-like containers sequentially.

14. Device according to one of claims 1 to 13, **characterised in that** the electrical signal-generating device is connected to a preferably programmable time-control unit, which is designed for the predetermined adjustment of operating times of the electrical signal-generating device.

## Revendications

1. Dispositif pour le traitement antibactérien, en particulier la décontamination et/ou la stérilisation de l'eau ainsi que pour la suppression des micro-organismes dans l'eau, avec
- un récipient (10) conçu pour recevoir une quantité d'eau prévue pour le traitement, et
- un agencement d'électrodes (12), conçu pour soumettre la quantité d'eau dans le récipient à un traitement, et susceptible d'être raccordé et de fonctionner avec un organe de production de signaux électriques (14), prévu à l'extérieur du récipient, dans lequel l'organe de production de signaux électriques (14) peut fonctionner à basse tension et est conçu pour produire un signal électrique alternatif entre les électrodes de l'agencement d'électrodes avec une amplitude maximale < 50 V et une fréquence de signal dans la plage située entre 1 et 5000 kHz, en particulier de 5 à 50 kHz, l'organe de production de signaux présente des moyens de réglage, qui sont conçus pour la modification automatique d'une amplitude maximale, d'un débattement d'amplitude et/ou d'un rapport signal/pauses du signal alternatif, en fonction d'une conductance de la quantité d'eau, le récipient et l'organe de production de signaux forment une unité portable et l'agencement d'électrodes comporte comme électrode (12), au moins une pièce conductrice, allongée au moins par tronçons, en forme de fil ou de barre ou configurée à titre d'échantillonnage sur une paroi interne du récipient.

2. Dispositif selon la revendication 1, **caractérisé en ce que** le signal électrique alternatif possède une composante de tension continue et est de préférence un signal de tension alternative redressé.

3. Dispositif selon la revendication 1 ou 2, **caractérisé en ce que** le récipient et l'organe de production de signaux forment une unité maniable manuellement.

4. Dispositif selon l'une des revendications 1 à 3, **caractérisé en ce que** l'agencement d'électrodes présente des électrodes en forme de fil d'un diamètre maximal de 0,5 mm, de préférence de 0,1 mm.

5. Dispositif selon l'une des revendications 1 à 4, **caractérisé en ce que** l'agencement d'électrodes est réalisé en un matériau de magnésium de manière que, en particulier en cas d'une teneur en chlorure présente dans la quantité d'eau prévue pour le traitement, des ions magnésium puissent entrer dans la quantité d'eau au cours du traitement.

6. Dispositif selon l'une des revendications 1 à 5, **caractérisé en ce que** les moyens de réglage sont conçus de sorte que, en cas de faible conductance de la quantité d'eau, l'amplitude de tension du signal alternatif soit augmentée et en cas de conductance élevée, l'amplitude de tension soit réduite.

7. Dispositif selon l'une des revendications 1 à 6, **caractérisé en ce que** les moyens de réglage sont conçus de sorte que, en cas de faible conductance de la quantité d'eau, l'intervalle de temps entre les impulsions successives du signal alternatif est réglé à une valeur basse, et en cas de conductance plus élevée, l'intervalle de temps est réglé à une valeur supérieure.

8. Dispositif selon l'une des revendications 1 à 7, **caractérisé par** une unité de filtrage montée en amont d'une entrée du récipient et/ou montée en aval d'une sortie du récipient.

9. Dispositif selon l'une des revendications 1 à 8, **caractérisé en ce que** le récipient est du moins partiellement rempli d'un matériau de filtrage et agit lui-même comme filtre, l'agencement d'électrodes prévu dans le récipient agissant en vue d'opérer la décontamination ou bien la stérilisation du récipient agissant comme filtre.

10. Dispositif selon l'une des revendications 1 à 9, **caractérisé en ce que** le récipient est conçu sous la forme d'un récipient de passage d'écoulement.

11. Dispositif selon l'une des revendications 1 à 10, **caractérisé par** des moyens pour la détermination électronique d'une conductance électrique de la quantité d'eau, les moyens étant raccordés à l'organe de production de signaux de manière que l'organe de production de signaux règle et émette, en réponse à une conductance prédéterminée, un modèle de signal associé du signal électrique alternatif.

12. Dispositif selon l'une des revendications 1 à 11, **caractérisé par** un agencement d'électrodes supplémentaire pouvant être soumis à une tension continue ou alternative applicable séparément, qui comprend du carbone, du magnésium et/ou un métal précieux et est disposé de manière que, grâce à son action, des oxydants anodiques, en particulier le chlore libre et les composés chlorés, dans la quantité d'eau soient neutralisables ou bien éliminables.

13. Dispositif selon l'une des revendications 1 à 12, **caractérisé en ce qu'**une pluralité de récipients, de préférence carrés, sont prévus de façon modulaire, et sont configurables de sorte que la quantité d'eau prévue pour le traitement puisse s'écouler simultanément à travers une pluralité de récipients de type modulaire, ou bien puisse s'écouler séquentiellement à travers la pluralité de récipients de type modulaire.

14. Dispositif selon l'une des revendications 1 à 13, **caractérisé en ce que** l'organe de production de signaux électriques est relié à une unité de synchronisation, de préférence programmable, qui est conçue pour le réglage prédéterminé des instants de fonctionnement de l'organe de production de signaux électriques.
